(19) Europäisches Patentamt
European Patent Office
Office européen des brevets

(11) Publication number: **0 231 608**
**A1**

# (12) EUROPEAN PATENT APPLICATION

(21) Application number: 86309272.2

(22) Date of filing: 27.11.86

(51) Int.Cl.$^3$: C 12 N 15/00
C 12 Q 1/68

(30) Priority: 28.11.85 GB 8529275

(43) Date of publication of application:
12.08.87 Bulletin 87/33

(84) Designated Contracting States:
AT BE CH DE ES FR GB GR IT LI LU NL SE

(71) Applicant: WHITBREAD & COMPANY PLC
Brewery Chiswell Street
London EC1Y 4SD(GB)

(72) Inventor: Lancashire, William Edward
21 Beaumont Green
Groby Leicestershire LE6 0EP(GB)

(72) Inventor: Hadfield, Christopher
13 Ashby Rise
Great Glen, Leicester LE8 0GB(GB)

(74) Representative: Perry, Robert Edward et al,
GILL JENNINGS & EVERY 53-64 Chancery Lane
London WC2A 1HN(GB)

## (54) DNA recombination processes and products.

(57) A process for modifying the genome of a micro-organism by integrating therein a DNA sequence of at least 15 bases, characterised in that the integrated sequence does not include a gene and in that the number of different essential genes in the genome, and the functions of the proteins which can be expressed by these genes, are unchanged by the modification.

The fact that the integrated sequence is "silent" allows the provenance of a micro-organism thus modified to be detected by the modifier only.

EP 0 231 608 A1

Croydon Printing Company Ltd

# DNA RECOMBINATION PROCESSES AND PRODUCTS

## FIELD OF THE INVENTION

This invention relates to DNA recombination processes, and to products obtained by such processes. In particular, it relates to integration techniques performed on micro-organisms, to plasmids used as starting materials in the procedures, and to a general method for identifying the provenance of micro-organisms.

## BACKGROUND OF THE INVENTION

Integration and transformation procedures are well known. For example, it is well known to transform a micro-organism such as a yeast with a foreign genomic fragment (modified as necessary to allow transformation), in order to introduce a foreign gene into the micro-organism. This technique has the object of producing the foreign protein by culturing the micro-organism.

The various integration procedures which are known have had the object of modifying the, say, natural genome in order to introduce a new function or to improve an existing function. In the case of pathogenic viruses, it is also known to introduce a deletion, with the object of inactivating a gene encoding a protein in the pathogenic pathway.

The transformation of yeasts by integration is a comparatively rare event if circular plasmids are used, see Kingsman et al (1985), Biotech. and Gen. Eng. Rev. 3, 377-416. Frequencies can be improved by transforming with a linear DNA fragment; see Orr-Weaver et al, (1981), Proc. Natl. Acad. Sci. USA 78, 6354-6358.

## ART PUBLISHED PRIOR TO THE CLAIMED PRIORITY DATE

Post and Roizman, Cell 25 (1981), 227-232, describe a general method for the inactivation and deletion of genes at specific sites in large DNA genomes. In the first step of the procedure, the Herpes simplex virus

thymidine kinase gene is inserted into the genome at a specific site. In the second step, the thymidine kinase gene and desired sequences flanking the insertion site are deleted. Both steps involve recombination of the genomes with cloned chimeric fragments, and utilise the available selection for or against thymidine kinase to select the desired genomes. Post and Roizman exemplify their technique by inactivating and deleting portions of an alpha gene of Herpes simplex virus I specifying protein 22. The recombinant virus carrying the thymidine kinase inserted into the gene 22, and viruses exhibiting deletions in this gene specify new alpha polypeptides.

The article by Post and Roizman is generally directed to the construction of a deletion in a functional gene, inactivating the gene and thereby changing the function of the genome. In the specific example, a new function is introduced. Post and Roizman also suggest that the principles of their method for transforming the natural genome with a marker, selecting the transvected virus and inserting the new function into the selected genome, "could be extended to construct insertions and deletions in cellular genomes".

Lee _et al_ (1985), Nucleic Acids Research _12_, No. 17, 6797-6812, disclose the integration of a duplicate genomic fragment into a plasmid, in order to increase production of a protein expressed by the gene. A circular plasmid designated pNL002 is shown in Fig. 1 (on page 6801) of the article, which includes two _Hind_III fragments in tandem. Fig. 3 (on page 6802) of the article shows in a degree of detail the "Intercistronic region between two IFN-B' genes tandemly arranged in accordance with the processes of Figs. 1 and 4-5".

ART PUBLISHED AFTER THE CLAIMED PRIORITY DATE

EP-A-0163491, published 4th December 1985 (corresponding to US Patent Application Serial No.

612,796, filed 22nd May 1984), discloses a genome, in a yeast, which has duplicate target sequences separated by a selectable marker, and in which one of the target sequences has inserted therein any gene or DNA sequence to be expressed in yeast. The genome including the duplicate target sequences is apparently considered to be inherently unstable, and construction of the modified genome is followed, in yeast, by a "kick-out" leaving a single target sequence including the inserted sequence, while losing marker sequences between the duplicate target sequences.

OBJECTS OF THE INVENTION

The present invention shares with Post *et al* and EP-A-0163491 the object of modifying the genome of a micro-organism by a procedure in which a dominant marker is first inserted into the unmodified genome, and the micro-organism modified by the insertion of the marker is selected before the marker is lost in the ultimately modified genome. Loss of the marker again allows selection. However, the present invention has as its object the insertion of a DNA sequence which does not change the essential genetic information.

It is a further object of the present invention to provide micro-organisms which include genomic information which is not readily apparent, i.e. which is known only to a modifier of the genome who deliberately inserts a suitable sequence, and who is thus able to identify the provenance of the same or a derived genome.

SUMMARY OF THE INVENTION

According to one aspect of the present invention, a process for modifying the genome of a micro-organism comprises integrating therein a DNA sequence of at least 15 bases, characterised in that the integrated sequence does not include a gene and in that the number of different essential genes in the genome, and the

functions of the proteins which can be expressed by these genes, are unchanged by the modification.

According to further aspects of the present invention, novel plasmid vectors have the structures pXYZ, pZYX and pZYX' as shown in the accompanying drawings.

BRIEF DESCRIPTION OF THE DRAWINGS

Figures 1 and 2 set out the steps of different processes for integrating a DNA sequence Y into the genome, having a target sequence XZ, of a micro-organism.

Figures 3, 4, 5 and 6 show respective plasmid vectors which can be used for the production of DNA fragments which can be used in the process of the present invention.

Figure 7 shows materials and process steps within the general scope of those shown in Figure 1, the specific materials being those used in the illustrative Example (below).

DEFINITIONS

The symbols used in Figures 1 and 2 have the following meanings:

X and Z are DNA sequences which, in tandem, define a fragment of the genome of a micro-organism to be modified by a process according to the present invention;

Y is a DNA sequence, of at least 15 bases, to be integrated into the unmodified genome;

M is a DNA sequence containing one or more dominant markers;

$R^1$ and $R^2$ indicate sites for different restriction endonucleases; and

--- represents continuation of the chromosome.

With respect to Figures 3 to 6, the abbreviations used are conventional and/or the following abbreviations apply:

pADC1 = promoter from yeast ADC1 gene

tCYC1 = terminator region from yeast CYC1 gene

$Ap^R$ = β-lactamase gene

CAT = chloramphenicol transacetylase gene

LacZ = β-galactosidase gene

In Figure 7, the dotted line again means the continuation of the chromosome, while "Bam" and "X/S" are used as abbreviations for "BamHI" and "XhoI/SalI", respectively.

DETAILED DESCRIPTION OF THE INVENTION

The processes shown in Figures 1 and 2 each comprise (1) a primary transformation event and (2) a secondary transformation event. The two processes are most obviously distinguished by the different structures of the respective modified genomes; the process of Figure 2 gives rise to duplicate sequences XZ and will be referred to herein as the "duplicative" event. The process of Figure 1 will be referred to herein as the "replacement" event.

With particular reference first of all to the replacement event and Figure 1, it may be seen that the first transformation event comprises inserting a dominant marker into the "target" site (XZ) within the unmodified genome. The targeting fragment, i.e. a fragment to replace XZ at the target site, is formed by cloning the dominant marker sequence M into the target nuclear DNA fragment (XZ) on a shuttle-plasmid vector pXMZ. The targeting fragment is released from the vector as a linear fragment by digestion with the restriction endonuclease enzyme $R^1$. The released fragment is used to transform the host by integrative recombination. This results in a primary transformant in which the target sequence has been replaced by a copy of the same DNA sequence within which the dominant marker is inserted. The presence of the dominant marker allows for the selection of transformant clones.

In the second transformation event, the targeted DNA region, marked with the dominant marker, is replaced by a second modified copy of the target DNA fragment. This second modified copy contains, rather than the dominant marker, the sequence (Y) to be inserted into the genome. This second modified fragment is released from the plasmid vector pXYZ by digestion with restriction endonuclease $R^1$, provided of course that there is no site for $R^1$ within the fragment XYZ. By linear integrative transformation with the fragment XYZ, the marked DNA target region undergoes gene replacement. Secondary transformants, i.e. the desired product, can be detected owing to loss of the marker.

The duplicative event shown in Figure 2 follows the same overall pattern as the replacement event. However, instead of using fragments XMZ and XYZ in the primary and secondary transformations, respectively, fragments ZMX and ZYX are used instead. Linear integrative transformation in each case gives the duplication which is shown. Once again, primary transformants can be selected owing the presence of the dominant marker; secondary transformants can be detected following its loss.

In general terms, it is easier to construct plasmids of the type pXMZ and pXYZ than those illustrated for use in the duplicative event. The replacement event may therefore be preferred for the modification of a genome in which, in the target sequence XZ, a gene is contained within X and/or within Z.

In general terms, it is preferable to avoid separation of X and Z if the fragment XZ includes a complete gene which is partly in each of sequences X and Z. In this case, the duplicative event may be preferred, if this would retain the function of the gene. It should be borne in mind that the product of the duplicative

event may be inherently unstable; the presence of the duplicate sequences XZ may in theory lead to "kick-out" of the fragment ZYX, and thus the loss of the desired insert. However, instability need not always be a problem.

There are certain cases in which the "separation" of a complete gene partly in X and partly in Y may not be a disadvantage. For example, XYZ may encode a protein different from XZ, but the function of the protein may be unchanged; for example, it is well known that the enzymatic function of, say, β-galactosidase is unaffected if the length of the protein is increased, provided that the enzymatic active site is unmodified. Further, the inactivation of a gene in sequence XZ by the insertion of Y may not be important if the genome contains more than one of the same gene, so that at least one gene is unaffected. Moreover, the effective destruction of a target gene which encodes an essentially inactive protein will not affect the essential function of the micro-organism containing the modified genome.

In summary, the inserted sequence Y is characterised in that there is no, or essentially no, difference between the functions of proteins expressed by the modified and the unmodified micro-organisms. In other words, the sequences XZ and XYZ should encode the same protein or proteins having the same function. By contrast with the usual desire to modify a genome in order to encode a foreign protein, the insert Y used in the present invention is essentially "silent".

In general terms, sequence Y could be a functional gene, part of a gene, or a chemically-synthetised nucleotide. However, if the sequence is known only to the modifier, and is "silent" so that its insertion is neither apparent to the user or even to the molecular analyst, the present invention allows a valuable

industrial organism to be labelled or "tagged". It is a particular feature of the present invention that, following a process of the type described above, the same or any other selected micro-organism can be tested, in order to identify its provenance, by determining whether the selected micro-organism includes the sequence Y.

Depending on the function of the inserted sequence Y, its construction may be made known or retained as a secret (for subsequent detection). For "tagging" purposes, sequence Y desirably contains a sufficient number of bases, e.g. at least 15, to provide a very low chance of random duplication in a micro-organism from a source other than the modifier. In practice, the genome of the micro-organism will be checked to ensure that it is free of any such duplication before sequence Y is introduced.

In general, individual steps of processes according to the invention (including integrative transformation and selection) can be conducted by standard techniques. Plasmids can be constructed by known techniques. Each plasmid may be stabilised in a suitable host, if necessary or desired, by including the necessary origin of replication. Detection of sequence Y can utilise a molecular probe device.

The invention can be used to modify the genome of any micro-organism, provided that a suitable target sequence (XZ) can be identified. The invention may be used to modify a naturally-occurring or genetically-engineered genome, e.g. of a micro-organism which has already been adapted to the large-scale production of a foreign protein. A particularly suitable micro-organism for use in the present invention is yeast, e.g. *Saccharomyces cerevisiae*.

The dominant marker in sequence M is, for example, $Cm^R$ (Hadfield, Cashmore and Meacock, abs. CSH Meeting

1985, p.57); the primary transformants may then be identified by chloramphenicol resistance. An alternative or additional screenable marker is lacZ (β-galactosidase) which allows identification by forming blue colonies on suitable respective growth media. Alternative markers, e.g. for yeast, are copper-resistance (Fogel and Welch, PNAS 79 (1982) 5342-5346) and G418-resistance (Hadfield, Cashmore and Meacock, abs. CSH Meeting 1985, p.58).

EXAMPLE

The present invention is illustrated, in this Example, by the introduction of new genetic material into the genome of *Saccharomyces cerevisiae*, following the scheme illustrated in Figure 7.

Plasmid Constructions

The successive integration process requires the use of two plasmids. The first plasmid directs a selectable dominant marker to the target site and the second directs the desired sequence, in this case a molecular "tag", to the target site for the replacement event. In this Example, the two plasmids constructed are based on plasmid pYRG12 (Hadfield *et al* (1986), Gene 45, 149-158; see Figure 3 herein) which is a derivative of plasmid pUC19 (Norrander *et al* (1983), Gene 26, 101-106; Vierra *et al* (1982) Gene 19, 259-268) into which a BamHI restriction digest DNA fragment of *Saccharomyces cerevisiae* chromosome XV including the HIS3 gene, has been cloned.

Construction of Vector for Primary Yeast Transformation

For the primary yeast transformation, in which the target DNA sequence is marked with a selectable marker, it was necessary to construct an integrating plasmid vector in which the dominant marker for chloramphenicol resistance is cloned within the targeting DNA sequence which is a fragment containing the HIS3 gene.

Plasmid pCH110 (Hadfield *et al*, *supra*; see Figure 4 herein) was cleaved with restriction enzyme *Sal*I and the resulting fragment of approximately 1.85 kb, containing the CAT gene, was ligated into the site generated by cleavage of plasmid pYRG12 with restriction enzyme *Xho*I. The ligation mix was transformed into *E. coli* strain HB101, and $Cm^R$ transformants were selected. A clone containing a plasmid of the required construction was identified by screening mini-prep. DNA (Holmes and Quigley (1981), Anal. Biochem. 114, 193-197) of several colonies by restriction map analysis. The selected plasmid was named pJK103 (Figure 5).

Construction of Vector for Secondary Yeast Transformation

For the second transformation event, in which the targeted gene is replaced with the sequence to be introduced into the genome, it was necessary to construct a plasmid in which the sequence to be integrated (in this example a molecular "tag" consisting of a 60-mer synthetic oligonucleotide) was cloned within the targeting DNA sequence which also contains the HIS3 fragment.

The oligonucleotide "tag" was designed to have two different cohesive ends, these being the recognition sequences for restriction endonuclease enzymes *Sal*I and *Cla*I. It was synthetised on an Applied Biosystems 380B system. The "tag" was ligated into plasmid pYRG12 which had been cleaved with restriction enzymes *Xho*I and *Cla*I. The mixture was transformed into *E. coli* strain HB101. Recombinant clones were screened for the presence of inserted "tag" by restriction enzyme mapping using mini-prep. DNA samples (Holmes and Quigley, *supra*). Plasmid pJKT2 (Fig. 6; T = tag) had the required structure and therefore was chosen; the presence of the oligonucleotide "tag" was verified by the "dot-blot" hybridisation technique (Maniatis *et al*, Cold

Spring Harbor Manual, p 331) and by Southern hybridisation using kinase end-labelled oligonucleotide as the probe (Cashmore et al (1986), Molec. Gen. Genet. 203, 154-162).

Yeast Transformations

(1) Primary Yeast Transformation

Commercial brewing yeast strain Y1 (300 µl) was transformed with plasmid pJK103 (1.0 µg) which had been cleaved with restriction enzyme BamHI to release the fragment containing the chloramphenicol-resistance ($Cm^R$) marker. The mixture was plated on YEP medium (1.0% yeast extract, 1.0% bacteriological peptone) containing 2.0% ethanol and 0.5% chloramphenicol and solidified with 2.3% agar. On this medium, only yeast cells containing the $Cm^R$ marker can multiply to form identifiable colonies on the Petri dish.

Several positive clones were screened by the colony hybridisation technique (Grunstein and Hogness (1975), Proc. Natl. Acad. Sci., USA 72, 3961-3965) using a radioactively-labelled probe based on the CAT gene and prepared using the oligonucleotide primer procedure (Feinberg and Vogelstein (1983), Anal. Biochem. 137, 266-267) to detect for successful integration of the DNA fragment containing the $Cm^R$ marker into the nucleus of the strain. The correct location of the insertion into the HIS3 region target site was verified by Southern hybridisation of the labelled probe to digested cellular DNA isolated from the primary clone and electrophoresed on a 1.0% agarose gel.

The presence of the $Cm^R$ marker was also verified by assaying for the enzyme determined by the $Cm^R$ marker gene, namely chloramphenicol transacetylase (see the Table). The procedures are described by Hadfield et al (1981), Gene, in press, and are essentially as follows: Logarithmic growth phase liquid broth cultures of yeast

are broken by vortex-mixing with glass beads, and extracts clarified by centrifugation. The essential characteristic of the assay is the production, from 5,5'-dithiobis(2-nitrobenzoic acid), of 5-thio-2-nitrobenzoate. The production of the latter is monitored by the absorption change at 412 nm. One unit of activity is defined as 1 μmole of 5-thio-2-nitrobenzoate produced per minute per mg at 25 C.

(2) Secondary Yeast Transformation

The primary yeast transformant (Y1/P1) isolated as described above was subjected to the secondary cycle of transformation using the secondary plasmid pJKT2, which had been cleaved with restriction enzyme *Bam*HI to release the fragment containing the molecular tag. After transformation, yeast cells were plated on YEP medium containing 2.0% ethanol and allowed to grow to form colonies. These were then velveteen replica-plated on to YEP medium containing 2.0% ethanol plus 0.5% chloramphenicol. After 7 days growth at 27 C, putative secondary transformants were identified as colonies which do not grow on this test medium, i.e. they are sensitive to chloramphenicol. These would have resulted from the secondary chromosomal exchange event shown in Figure 7.

The loss of $Cm^R$ marker was verified by the CAT assay procedure, as described above. The results of the verification are given in the Table, below.

In order to verify the presence of the molecular "tag" in the transformants they were screened by the following three methods:

(a) Yeast colony hybridisation (Grunstein and Hogness, (1975), Proc. Natl. Acad. Sci. USA, 72, 3961) using the oligonucleotide probe labelled with $^{32}P$ by kinasing as described in Maniatis *et al* (1982), A Laboratory Manual CSH, p 123.

(b) Total cellular DNA was prepared from the yeast transformants (Cashmore et al, supra), digested with restriction enzyme BamHl, electrophoresed on 0.8% agarose and subjected to the Southern hybridisation technique (Southern (1975), J. Mol. Biol. 98, 503-517) using the above-mentioned probe.

(c) Total cellular DNA prepared as in (b) was probed with the above-mentioned probe, using the "Dot-blot" hybridisation procedure referred to earlier. DNA hybridisation with primary and secondary transformants was observed; the results are given in the following Table (which collates the distinguishing characteristics of parental, primary and secondary ("tagged") yeast strains).

| Yeast Strain | Growth on 0.5 mg/ml CAP | CAT Activity Units | Hybridisation reaction of genomic DNA with DNA probes | |
|---|---|---|---|---|
| | | | CAT probe | "TAG" sequence probe |
| Parental | - | 0 | - | - |
| Primary Integrant | + | 0.06 | + | - |
| Secondary Integrant | - | 0 | - | + |

Secondary transformant Y1/T5 was chosen for further study in pilot brewery trials. Y1/T5 was deposited at the National Collection of Yeast Cultures, Colney Lane, Norwich, England, on 14th November 1986. Its accession number is NCYC 1602.

Pilot Scale Ale Fermentation

Pilot scale fermentations were carried out in cylindro-conical fermenter vessels, maximum capacity 12 barrels (approx. 2000 l), in the pilot brewery at Whitbread Scientific Services Department, Whitbread Breweries, Oakley Road, Luton, England.

Pre-cultures of "tagged" yeast strain Y1/T5 for the fermentations were grown in aerated YPD broth. 400 ml shaken cultures thus produced were inoculated into 40 litre cultures of aerated brewers wort. After aerated growth, these were used to inoculate the main culture.

Brewer's wort (ale) was prepared from 87.5% ale malt, 5.0% crystal malt and 7.5% torrified wheat with the addition of hops (to 31 EBU bitterness units) and caramel. After boiling and submitting to a whirlpool stand, the wort was transferred to the fermenter. The specific gravity at collection was 1.044.

Yeast was pitched (2 g pressed yeast/litre) into the fermenter containing approximately 1,000 litres of brewers' wort. The maximum fermentation temperature was 20 C. Samples were taken twice daily for analysis of gravity, total suspended material and stability of the integrated sequence. After 72 hours (specific gravity 1.010), the fermentor was chilled to 15 C and the beer was racked and transferred to a collection tank where finings were added. The beer was left to condition for 1 week at 0 C. The conditioned beer was then filtered and diluted to correspond to an original gravity of 1.037.

The above pilot brewery procedure was repeated for the parental control strain Y1.

The rate of drop of specific gravity of the fermenting worts shows that the "tagging" procedure does not affect the performance of the strain in terms of rate of fermentation. There was no significant difference in size of the final yeast crop or yeast viability.

Samples of beer were subsequently packaged and pasteurised. The bottled samples were then subjected to the usual analytical tests, including analysis of foam and head properties, flavour compounds, bitterness and standard flavour trials. No significant differences were observed between the beer produced by the tagged strain and that produced by the standard parent strain.

CLAIMS

1. A process for modifying the genome of a micro-organism by integrating therein a DNA sequence of at least 15 bases, characterised in that the integrated sequence does not include a gene and in that the number of different essential genes in the genome, and the functions of the proteins which can be expressed by these genes, are unchanged by the modification.

2. A process for modifying the genome of a micro-organism, such that the modified genome includes the DNA sequence

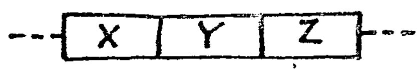


while the corresponding unmodified sequence is

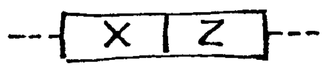


wherein sequences X and Z in tandem define a fragment of the unmodified genome; Y is an integrated sequence, of at least 15 bases, which is characterised in that there is no, or essentially no, difference between the functions of proteins expressed by the modified and the unmodified micro-organisms; and --- indicates continuation of the chromosome; which comprises the steps of:

(1) transforming the unmodified micro-organism with a marked DNA fragment of the structure

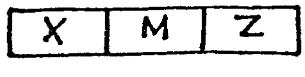


wherein sequence M includes a dominant marker, and selecting the resultant marked micro-organism which includes the sequence

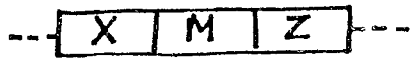


and

(2) transforming the selected micro-organism

including the dominant marker with a DNA fragment of the structure

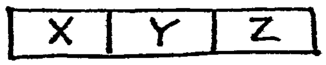


and selecting the resultant micro-organism from which the dominant marker is absent.

3. A process according to claim 2, wherein the marked fragment XMZ is obtained by using a restriction endonuclease ($R^1$) to digest a circular plasmid vector having the structure

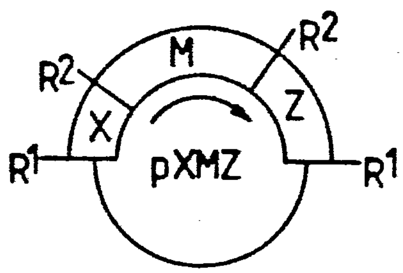


wherein $R^1$ and $R^2$ indicate sites for different restriction endonucleases, there being no site for $R^1$ within the fragment.

4. A circular plasmid vector having the structure

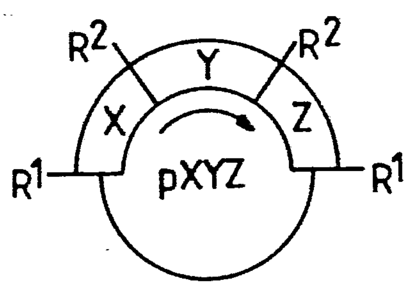

wherein $R^1$ and $R^2$ indicate sites for different restriction endonucleases, there being no site for $R^1$ within the fragment XYZ; X and Z in tandem define a genomic fragment of a micro-organism; and Y is an integrated sequence, of at least 15 bases, which is characterised in that the sequences XZ and XYZ encode the same protein or proteins having the same function.

5. A process according to claim 2 or claim 3, wherein the fragment XYZ is obtained by digesting the circular plasmid vector defined in claim 4 with the restriction endonuclease $R^1$.

6. A process for modifying the genome of a micro-organism, such that the modified genome includes the DNA sequence

| --- | X | Z | Y | X | Z | --- |
|---|---|---|---|---|---|---|

wherein X, Y, Z, --- and the unmodified sequence are as defined in claim 2, which comprises the steps:

(1) transforming the unmodified micro-organism with a marked DNA fragment of the structure

| Z | M | X |
|---|---|---|

wherein sequence M includes a dominant marker, and selecting the resultant marked micro-organism which includes the sequence

| --- | X | Z | M | X | Z | --- |
|---|---|---|---|---|---|---|

and

(2) transforming the selected micro-organism including the dominant marker with a DNA fragment of the structure

| Z | Y | X |
|---|---|---|

and selecting the resultant micro-organism from which the dominant marker is absent.

7. A process according to claim 6, wherein the marked fragment ZMX is obtained by using a restriction endonuclease ($R^2$) to digest a circular plasmid vector having the structure

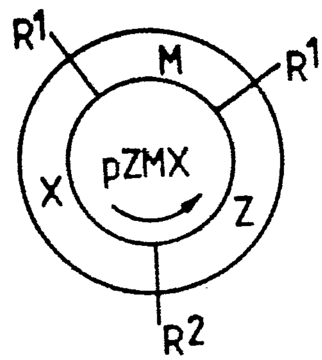


wherein $R^1$ and $R^2$ indicate sites for the different restriction endonucleases, the site indicated by $R^2$ being unique in the plasmid.

8. A circular plasmid vector having the structure

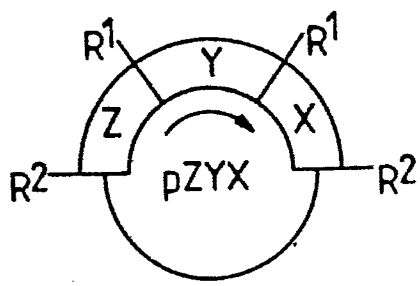


wherein $R^1$ and $R^2$ indicate sites for different restriction endonucleases, there being no site for $R^2$ within the fragment ZYX; X and Z in tandem define a genomic fragment of a micro-organism; and Y is an integrated sequence, of at least 15 bases, which is characterised in that the modified and unmodified genomes encode the same protein or proteins having the same function.

9. A circular plasmid vector having the structure

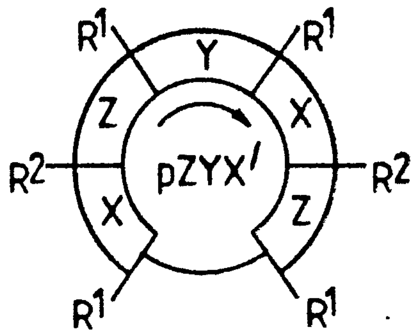


.

wherein $R^1$ and $R^2$ indicate sites for different restriction endonucleases, there being no site for $R^2$ within the fragment ZYX; X and Z in tandem define a genomic fragment of a micro-organism; and Y is an integrated sequence, of at least 15 bases, which is characterised in that the modified and unmodified genomes encode the same protein or proteins having the same function.

10. A process according to claim 6 or claim 7, wherein the fragment ZYX is obtained by digesting the circular plasmid vector defined in claim 8 or claim 9 with the restriction endonuclease $R^2$.

11. A process according to any of claims 1, 2, 3, 5, 6, 7 and 10, wherein either or each of sequences X and Z includes a complete gene, and there is no difference between the proteins expressed by the modified and the unmodified micro-organisms.

12. A process according to any of claims 6, 7 and 10, wherein the fragment XZ includes a complete gene which is partly in each of sequences X and Z.

13 A method for identifying the provenance of a selected micro-organism, which comprises conducting a process according to any preceding claim, and subsquently testing the selected micro-organism to determine whether it includes the integrated sequence.

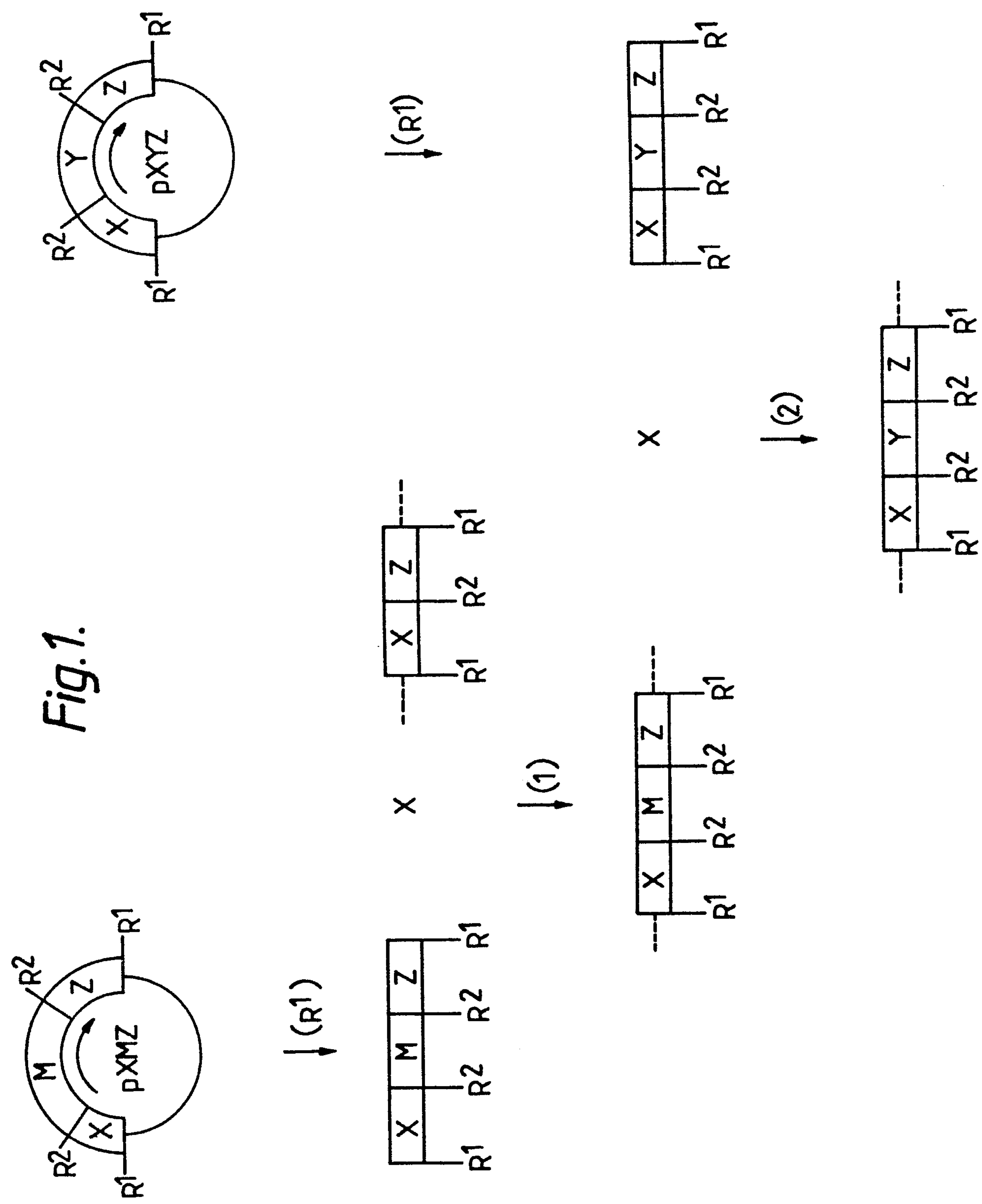

Fig.1.

Fig.2.

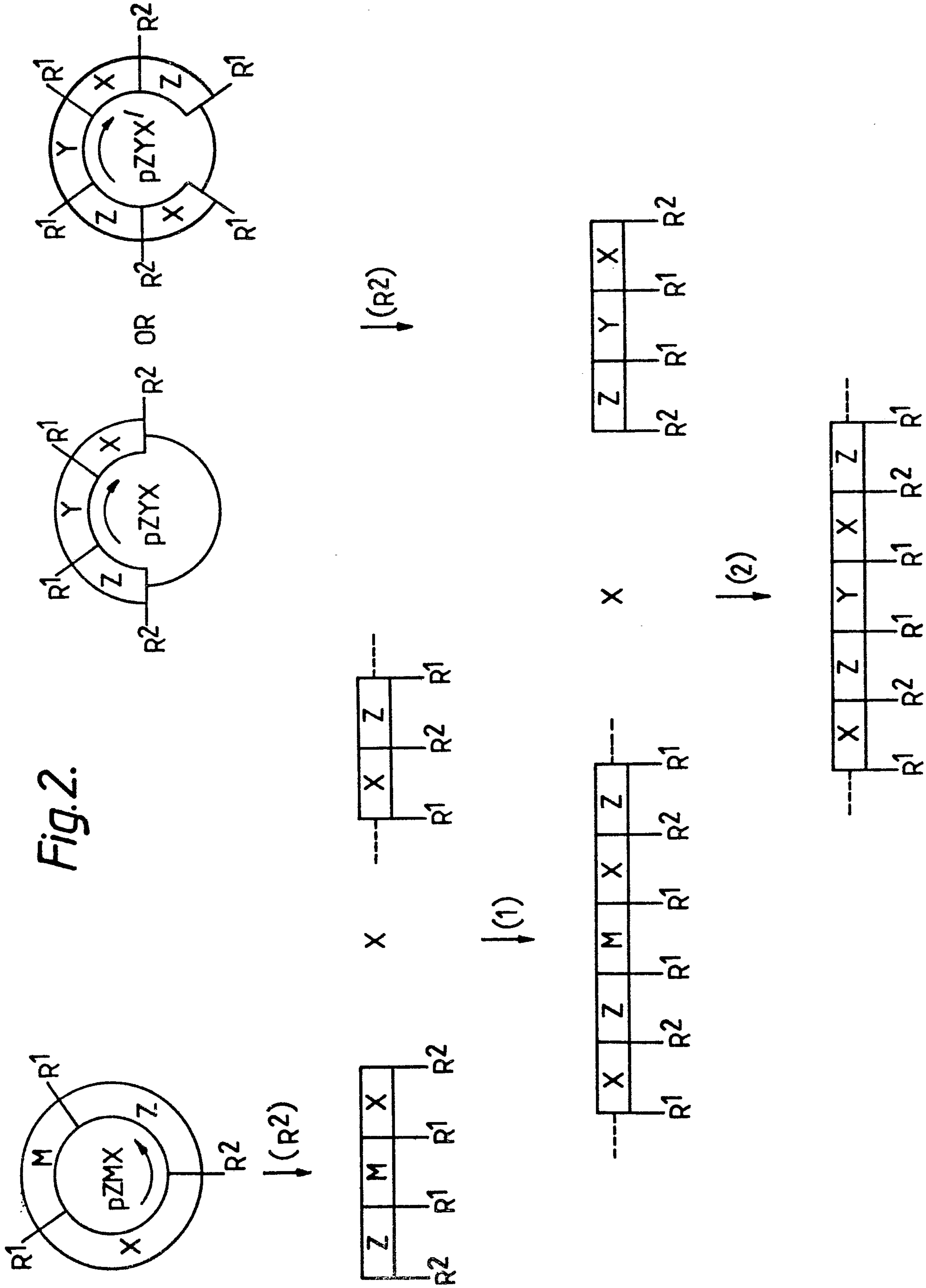

Fig.3.

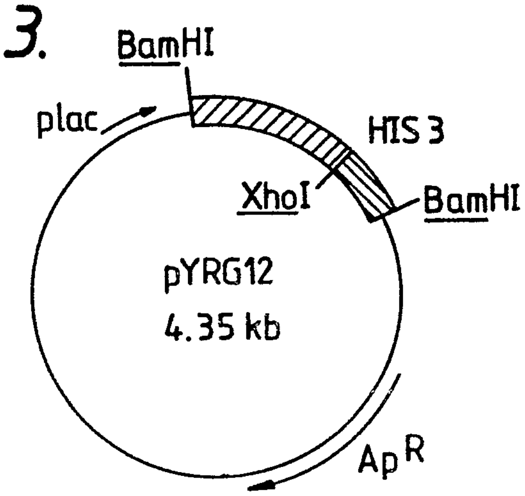


Fig.4.

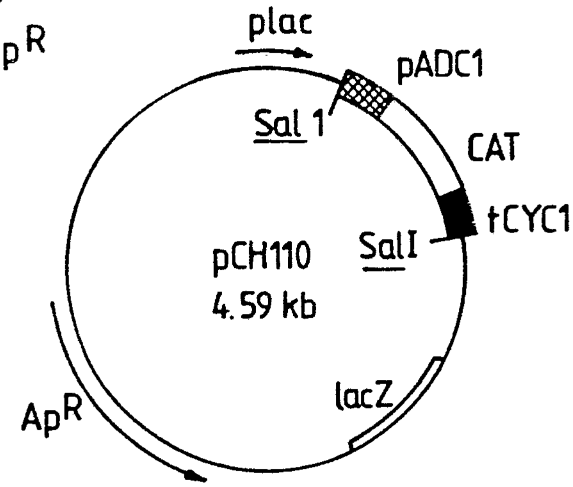


Fig.5.

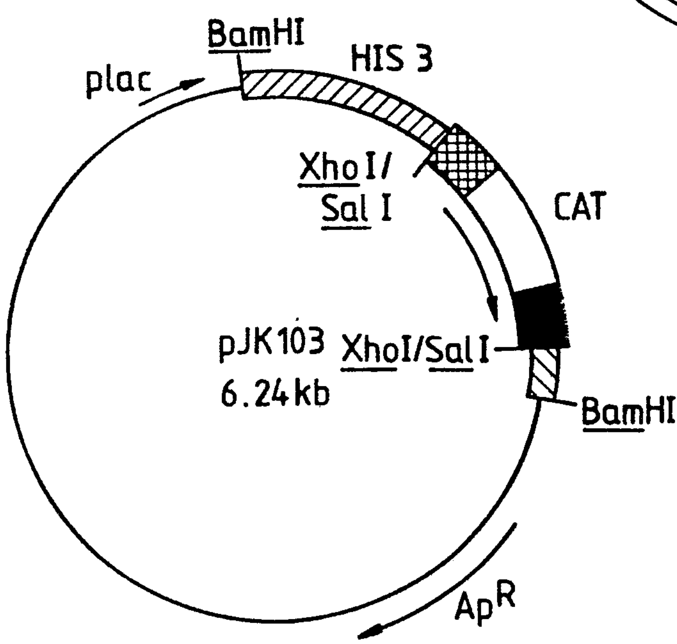


Fig.6.

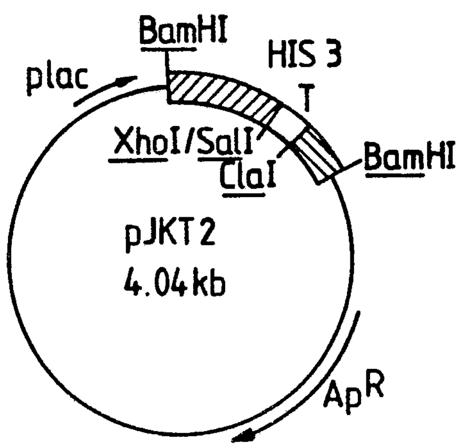

Fig.7.

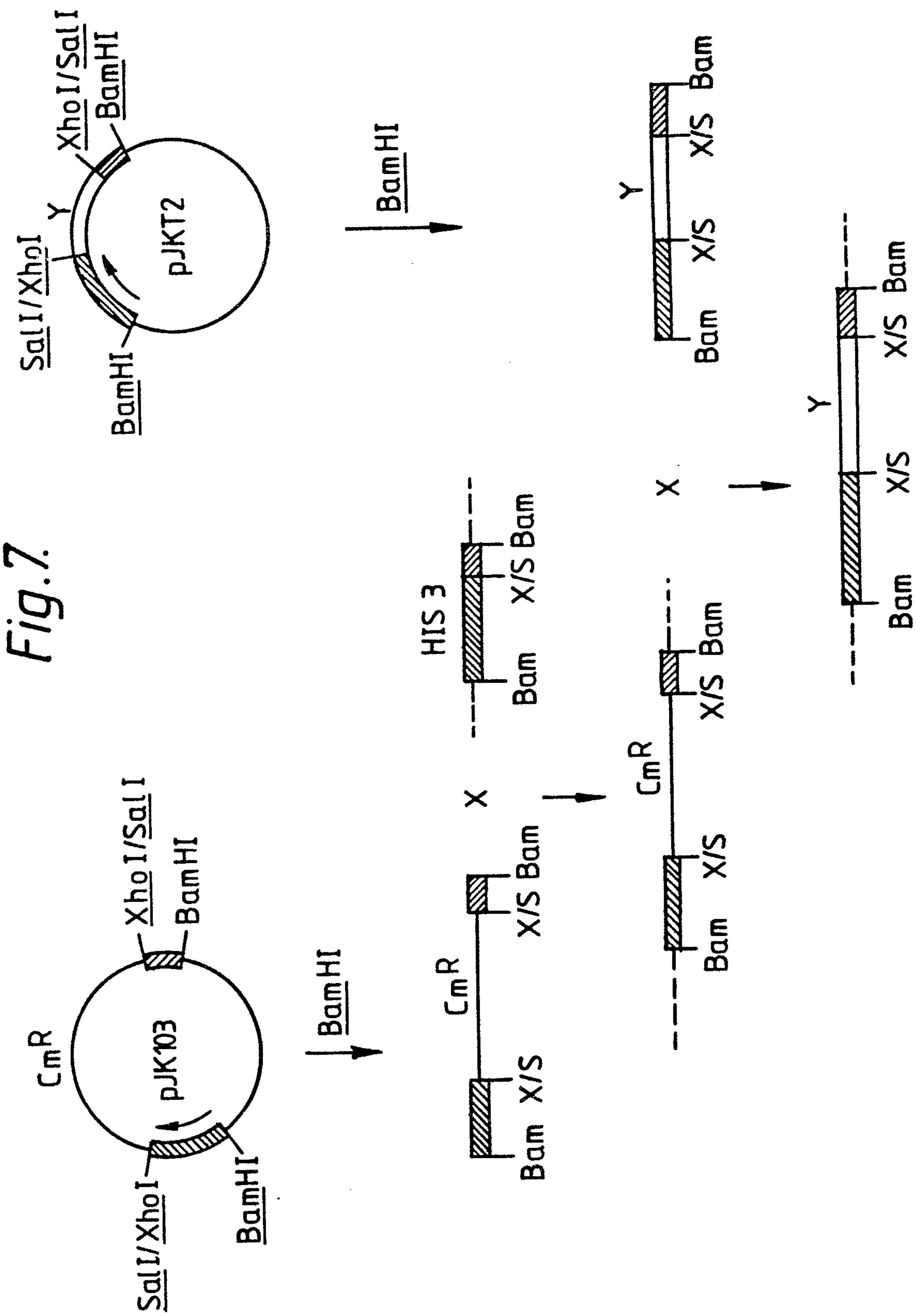

European Patent Office

# EUROPEAN SEARCH REPORT

0231608
Application number

EP 86 30 9272

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| A | EP-A-0 138 508 (PFIZER INC.)<br>* Pages 35-42 *<br>--- | 1-7 | C 12 N 15/00<br>C 12 Q 1/68 |
| A,D | CELL, vol. 25, July 1981, pages 227-235, MIT; L.E. POST et al.: "A generalized technique for deletion of specific genes in large genomes: alpha gene 22 of herpes simplex virus 1 is not essential for growth"<br>* Whole document *<br>--- | 1-7 | |
| A,P D | EP-A-0 163 491 (BIOTECHNICA INTERNATIONAL, INC.)<br>* Whole document *<br>----- | 1-7 | |
| | | | TECHNICAL FIELDS SEARCHED (Int Cl.4) |
| | | | C 12 N<br>C 12 Q |

The present search report has been drawn up for all claims

| Place of search | Date of completion of the search | Examiner |
|---|---|---|
| THE HAGUE | 06-03-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document